# FASCICULE DE BREVET EUROPEEN

(11) **EP 1 263 406 B1**
(45) Date de publication et mention de la délivrance du brevet: **15.09.2004**
(21) Numéro de dépôt: 01913982.3
(22) Date de dépôt: 12.03.2001
(51) Int. Cl.: A61K 7/48

(54) **MELANGE D'ESTERS GRAS POUR LA PROTECTION SOLAIRE ET PRODUIT COSMETIQUE EN CONTENANT**
EINE MISCHUNG VON FETTSÄUREESTERN ZUR SONNENSCHUTZ UND LICHTSCHUTZFORMULIERUNGEN DIESE ENTHALTEND
FATTY ESTER MIXTURE FOR SOLAR PROTECTION AND COSMETIC PRODUCT CONTAINING SAME

(30) Priorité: 13.03.2000 FR 0003158
(43) Date de publication de la demande: 11.12.2002
(73) Titulaire: Stearinerie Dubois Fils, Societe Anonyme, 36300 le Blanc (FR)
(72) Inventeur: LEGE, Corinne, F-92000 Nanterre (FR); LOUBAT, Nathalie, F-92380 Garches (FR)
(74) Mandataire: Hammond, William
(86) Numéro de dépôt international: PCT/FR2001/000724
(87) Numéro de publication internationale: WO 2001/068050

(56) Documents cités:
- WO-A-95/00107
- FR-A- 2 789 308

## Description

La présente invention est relative à un mélange d'esters gras notamment pour produit cosmétique contenant au moins un filtre solaire, tel que par exemple un produit de protection solaire, ainsi qu'à un produit cosmétique comportant ce mélange d'esters gras.

Les produits cosmétiques destinés à la peau peuvent se présenter sous différentes formes dont, par exemple, des émulsions ou des huiles. Parmi ces produits,certains peuvent comprendre une combinaison de produits filtrant les ultraviolets.

Il sera rappelé que les produits filtrant les ultraviolets ont pour fonction de protéger la peau contre les coups de soleil dont la brûlure peut être d'un degré important.

Les mélanges connus d'esters gras, outre leur fonction de solubilisation des filtres solaires, jouent également un rôle pour favoriser l'étalement du produit cosmétique sur la peau afin d'en assurer une couverture homogène et protectrice. Les produits cosmétiques comprennent aussi des cires, des produits tensioactifs, des corps gras (autres que les esters gras) dont des huiles minérales ou végétales, des silicones, des parfums, des anti-oxydants, des principes actifs, des colorants, des pigments, des épaississants, des agents de texture, etc. et ce en des proportions variant notamment en fonction des qualités recherchées du produit cosmétique ainsi réalisé.

Un produit cosmétique peut être défini par son « facteur de protection solaire », ou SPF : la tendance actuelle, notamment dans les produits de protection solaire, est d'améliorer cette valeur.

Parmi les produits connus pouvant constituer un mélange de corps gras, on utilise beaucoup le benzoate de C₁₂-C₁₅ le plus souvent en association avec des silicones volatils.

Mais on connaît d'autres corps gras tels que ceux cités dans le document US-4 940 574-A qui sont tous connus pour leur pouvoir de solubilisation des produits filtrants et pour leur qualité quant à l'étalement du produit cosmétique.

Un des buts de la présente invention est de fournir un mélange d'esters gras qui présente les fonctions ci-dessus, mais qui aurait en plus une action favorable sur le facteur de protection solaire (SPF).

Un autre but est de fournir un tel mélange d'esters gras qui permet de préparer un produit cosmétique performant et à un coût économique raisonnable.

Ces buts, ainsi que d'autres qui apparaîtront par la suite, sont atteints par un mélange d'esters gras notamment pour produit cosmétique, qui est caractérisé, selon la présente invention, par le fait qu'il comprend au moins deux esters gras choisis parmi les esters d'un acide mono carboxylique ramifié en C₄-C₈ et d'un alcool ramifié en C₈-C₁₂, les esters d'un acide dicarboxylique linéaire en C₈-C₁₂ et d'un alcool ramifié en C₃-C₆ et les esters d'un acide hydroxycarboxylique et d'un alcool linéaire en C₁₀-C₁₈.

Avantageusement, parmi les esters d'un acide mono carboxylique ramifié en C₄-C₈ et d'un alcool ramifié en C₈-C₁₂ on choisit l'ester isodécylique de l'acide 2,2-diméthyl propionique.

De préférence, parmi les esters d'un acide dicarboxylique linéaire en C₈-C₁₂ et d'un alcool ramifié en C₃-C₆ on choisit l'ester isopropylique de l'acide décanedioïque.

Avantageusement, parmi les esters d'un acide hydroxycarboxylique et d'un alcool linéaire en C₁₀-C₁₈ on choisit l'ester alkylique en C₁₂-C₁₅ de l'acide 2-hydroxypropanoïque.

Selon un mode préféré de la présente invention, le mélange d'esters gras comprend au plus 75 % en poids de chacun de ces esters gras et, de préférence, en proportions sensiblement égales.

La présente invention concerne également un produit cosmétique comportant un mélange d'esters gras tel que défini ci-dessus.

La description qui va suivre et qui ne présente aucun caractère limitatif, permettra à l'homme du métier de mieux comprendre l'objet et avantages de la présente invention.

Un mélange d'esters gras pour un produit cosmétique comprend, selon la présente invention, au moins deux esters gras choisis parmi les esters d'un acide mono carboxylique ramifié en C₄-C₈ et d'un alcool ramifié en C₈-C₁₂, les esters d'un acide dicarboxylique linéaire en C₈-C₁₂ et d'un alcool ramifié en C₃-C₆ et les esters d'un acide hydroxycarboxylique et d'un alcool linéaire en C₁₀-C₁₈.

Il est en fait apparu de façon tout à fait surprenante, qu'une telle formulation d'un mélange d'esters gras permettait d'augmenter le facteur de protection solaire (SPF) sans pour autant augmenter la teneur en produits filtrants pour les ultraviolets, ainsi que le montre les exemples de réalisation ci-après :

### EXEMPLE 1

On réalise trois produits n°1, n°2 et n°3 de protection solaire comportant chacun en commun et dans les mêmes quantités :
- de l'éthanol,
- un produit filtrant les ultraviolets A tel que celui commercialisé par la société ROCHE sous la dénomination commerciale PARSOL 1789,
- deux produits filtrants les ultraviolets B tel que ceux commercialisés par la société BASF sous les dénominations commerciales UVINUL MBC95 et UVINUL T150.

Le produit n°1 comporte comme ester gras 70 % en poids de l'ester isodécylique de l'acide 2,2-diméthyl propionique, aussi dénommé néopentanoate d'isodécyle.

Le produit n°2 comporte comme ester gras 70% en poids de l'ester isopropylique de l'acide décanedioïque, aussi dénommé sébacate de di-isopropyle.

Le produit n°3 comporte 70 % en poids d'un mélange des deux esters gras précédents dans les proportions suivantes : 38 % en poids de néopentanoate d'isodécyle et 32 % en poids de sébacate de di-isopropyle.

La mesure du facteur de protection solaire (SPF) pour chacun de ces produits montre que celui du produit n°3 est augmenté de 30 % par rapport à celui du produit n°1 et de 25 % par rapport à celui du produit n°2.

### EXEMPLE 2

On réalise un produit n°4 comprenant, d'une part, les mêmes composants en commun que ceux des produits n°1, n°2 et n°3 de l'exemple 1 et, d'autre part, 70 % en poids d'un mélange d'esters gras constitué de 24% en poids de néopentanoate d'isodécyle, de 23 % en poids de sébacate de di-isopropyle et de 23 % en poids d'un ester alkylique en C₁₂-C₁₅ de l'acide 2-hydroxypropanoïque, aussi dénommé lactate de C₁₂-C₁₅.

La mesure du facteur de protection solaire (SPF) pour ce produit n°4 montre qu'il est augmenté de 5 % par rapport à celui du produit n°3.

Selon ces exemples, il apparaît que l'association des esters gras selon la présente invention et des produits filtrant les ultraviolets permet d'augmenter, de façon plus que notable, le facteur de protection solaire (SPF) du produit cosmétique ainsi obtenu : il existe donc une synergie.

## Revendications

1. Produit pour la protection solaire, **caractérisée par le fait qu'**il comprend, d'une part, un mélange d'au moins deux esters gras choisis parmi les esters d'un acide mono carboxylique ramifié en C₄-C₈ et d'un alcool ramifié en C₈-C₁₂, les esters d'un acide dicarboxylique linéaire en C₈-C₁₂ et d'un alcool ramifié en C₃-C₆ et les esters d'un acide hydroxycarboxylique et d'un alcool linéaire en C₁₀-C₁₈ et, d'autre part, un produit filtrant les ultra-violets.

2. Produit selon la revendication 1, **caractérisée par le fait que** parmi les esters d'un acide mono carboxylique ramifié en C₄-C₈ et d'un alcool ramifié en C₈-C₁₂ on choisit l'ester isodécylique de l'acide 2,2-diméthyl propionique.

3. Produit selon la revendication 1, **caractérisée par le fait que** parmi les esters d'un acide dicarboxylique linéaire en C₈-C₁₂ et d'un alcool ramifié en C₃-C₈ on choisit l'ester isopropylique de l'acide décanedioïque.

4. Produit selon la revendication 1, **caractérisée par le fait que** parmi les esters d'un acide hydroxycarboxylique et d'un alcool linéaire en C₁₀-C₁₈ on choisit l'ester alkylique en C₁₂-C₁₅ de l'acide 2-hydroxypropanoïque.

5. Produit selon l'une quelconque des revendications 1 à 4, **caractérisée par le fait qu'**il comprend au plus 75% en poids de chacun desdits esters.

6. Produit selon l'une quelconque des revendications 1 à 4, **caractérisée par le fait qu'**il comprend ces esters en proportions sensiblement égales.

7. Produit cosmétique pour la protection solaire, **caractérisé par le fait qu'**il comporte un produit selon l'une quelconque des revendications 1 à 6.

## Patentansprüche

1. Sonnenschutzprodukt, **dadurch gekennzeichnet, dass** es einerseits ein Gemisch von mindestens zwei Fettsäureestern, die unter den Estern einer verzweigten C₄-C₈-Monocarbonsäure und eines verzweigten C₈-C₁₂-Alkohols, den Estern einer linearen C₈-C₁₂-Dicarbonsäure und eines verzweigten C₃-C₆-Alkohols und den Estern einer Hydroxycarbonsäure und eines linearen C₁₀-C₁₈-Alkohols ausgewählt sind, und andererseits ein UV-Filtermittel enthalten.

2. Produkt nach Anspruch 1, **dadurch gekennzeichnet, dass** unter den Estern einer verzweigten C₄-C₈-Monocarbonsäure und eines verzweigten C₈-C₁₂-Alkohols der Isodecylester von 2,2-Dimethylpropionsäure ausgewählt ist.

3. Produkt nach Anspruch 1, **dadurch gekennzeichnet, dass** unter den Estern einer linearen C₈-C₁₂-Dicarbonsäure und eines verzweigten C₃-C₆-Alkohols der Isopropylester von Decandisäure ausgewählt ist.

4. Produkt nach Anspruch 1, **dadurch gekennzeichnet, dass** unter den Estern einer Hydroxycarbonsäure und eines C₁₀-C₁₈-linearen Alkohols ein C₁₂-C₁₅-Alkylester von 2-Hydroxypropansäure ausgewählt ist.

5. Produkt nach einem der Ansprüche 1 bis 4, **dadurch gekennzeichnet, dass** es höchstens 75 Gew.-% eines jeden dieser Ester enthält.

6. Produkt nach einem der Ansprüche 1 bis 4, **dadurch gekennzeichnet, dass** es diese Ester in im wesentlichen gleichen Anteilen enthält.

7. Kosmetisches Produkt für den Sonnenschutz, **dadurch gekennzeichnet, dass** es ein Produkt nach einem der Ansprüche 1 bis 6 enthält.

## Claims

1. A sun shield product **characterised in that** it comprises an ultra-violet filtering product and a mixture of at least two fatty acids chosen from among esters of a C₄-C₈ branched monocarboxylic acid and a C₈-C₁₂ branched alcohol, the esters of a C₈-C₁₂ straight-chain carboxylic acid and of a C₃-C₆ branched alcohol, and the esters of a hydroxycarboxylic acid and of a C₁₀-C₁₈ straight-chain alcohol.

2. A product according to claim 1, **characterised in that** the isodecyl ester of 2,2-dimethyl propionic acid is chosen from among esters of a C₄-C₈ branched monocarboxylic acid and a C₈-C₁₂ branched alcohol.

3. A product according to claim 1, **characterised in that** the isopropyl ester of decanedioic acid is chosen from among esters of a C₈-C₁₂ straight-chain dicarboxylic acid and a C₃-C₆ branched alcohol.

4. A product according to claim 1, **characterised in that** the C₁₂-C₁₅ alkyl ester of 2-hydroxypropanoic acid is chosen from among esters of a hydroxycarboxylic acid and a C₁₀-C₁₈ straight-chain alcohol.

5. A product according to any of claims 1 to 4, **characterised in that** it contains at most 75% by weight of each of the said esters.

6. A product according to any of claims 1 to 4, **characterised in that** it contains the said esters in substantially equal proportions.

7. A cosmetic product for protection against the sun, **characterised in that** it comprises a product of according to any of claims 1 to 6.
